# FASCICULE DE BREVET EUROPEEN

(11) **EP 0 565 489 B1**
(45) Date de publication et mention de la délivrance du brevet: **30.10.1996**
(21) Numéro de dépôt: 93810248.0
(22) Date de dépôt: 06.04.1993
(51) Int. Cl.: A63B 23/18

(54) **Appareil thérapeutique spécifique du domaine respiratoire**
Therapiegerät zur spezifischen Anwendung in Atmungsbereich
Therapeutic apparatus for specific use in the respiratory field

(30) Priorité: 10.04.1992 CH 1186/92
(43) Date de publication de la demande: 13.10.1993
(73) Titulaire: VARIORAW PERCUTIVE S.A., 1170 Aubonne (CH)
(72) Inventeur: Liardet, Claude, CH-1170 Aubonne (CH)
(74) Mandataire: Ganguillet, Cyril

(56) Documents cités:
- EP-A- 0 311 770
- EP-A- 0 411 714
- WO-A-89/03707
- US-A- 4 062 358
- US-A- 4 231 375
- US-A- 4 739 987

## Description

La présente invention a pour objet un appareil thérapeutique spécifique du domaine respiratoire.

Un appareil de résistance à l'expiration destiné à améliorer la ventilation pulmonaire est décrit dans le brevet européen no 0337990. Cet appareil est de petites dimensions, simple et peu coûteux. Il peut être facilement transporté, par exemple dans une poche, et permet d'obtenir des résultats équivalents à ceux obtenus avec les appareils de conception plus complexe, qui sont en général fort encombrants et fort coûteux. Il comprend une première partie de forme tubulaire comportant un orifice d'entrée d'air dans lequel le patient doit expirer et une seconde partie comportant un canal d'échappement de forme conique circulaire, dans lequel est disposée une bille sphérique de diamètre supérieur aux dimensions de l'orifice d'entrée du canal, de façon à obstruer le canal avant expiration. L'axe du canal conique est incliné vers le haut par rapport à l'axe de la partie de forme tubulaire d'un angle qui peut être compris entre 30° et 80°. L'angle formé par une génératrice de la paroi de ce canal et son axe est inférieur à l'angle d'inclinaison de l'axe du canal. L'appareil comprend en outre un ou plusieurs trous situés dans une zone opposée à l'orifice d'entrée du canal conique pour permettre à l'air expiré de s'échapper, les dimensions du trou étant choisies de façon à empêcher l'échappement de la bille. La bille peut ainsi se déplacer librement dans le canal en opposant une résistance à l'expiration de l'air expiré par le patient, la partie la plus basse du canal constituant un lit de roulement pour la bille, tandis que la partie la plus haute du canal constitue une butée au mouvement de la bille. Avant l'expiration, la bille obstrue le canal du cône. Durant l'expiration, sa position instantanée résulte d'un état d'équilibre entre la pression de l'air expiré et la force de gravité de la bille combinée avec la pente de son lit de roulement. Ainsi, pendant l'expiration, du fait de sa caractéristique d'amortissement très faible, la bille effectue un mouvement oscillatoire engendrant une pression variable qui s'oppose à l'expiration, en constituant une résistance oscillante positive à l'expiration.

L'expérience a montré que cet l'appareil permet une ventilation en percussion d'une grande efficacité, des sécrétions étant mobilisées en quelques minutes dans l'arbre bronchique du patient, permettant leur expectoration aisée. Des mesures par ballonnets oesophagiens ont montré que le phénomène de percussion atteint le niveau périphérique du poumon. Les études cliniques entreprises ont permis de mettre en évidence, une résistance oscillante à l'expiration COS (Controlled Oscillating System) de valeur toujours positive, adaptée aux valeurs thérapeutiques connues. Ces études ont également montré que le système COS permet une régulation automatique de la pression et une adaptation de la fréquence de l'oscillation, ces deux caractéristiques étant inhérentes au système et non dépendantes du débit d'air.

La synthèse des études cliniques mentionnées ci-dessus, complétée par des observations expérimentales très rigoureuses ont permis d'établir la courbe du cycle d'ouverture/fermeture de l'appareil décrit ci-dessus.

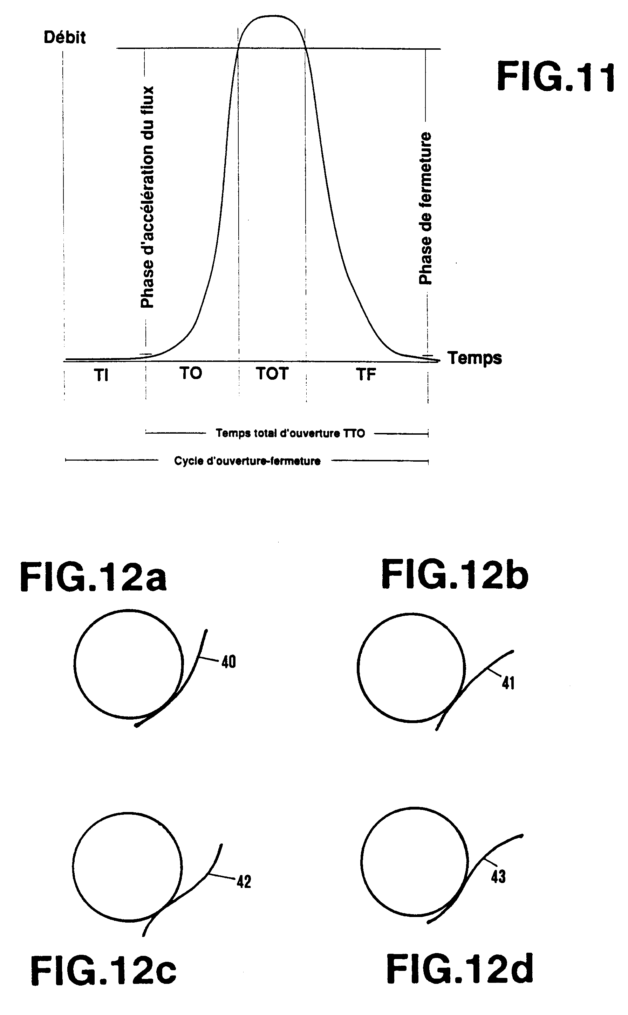

Cette courbe représentée à la figure 11 met en évidence le principe de fonctionnement de l'appareil. Après une surpression, la bille qui obstrue le canal se déplace et laisse l'air s'échapper par un orifice, croissant en fonction de sa position (la vitesse du flux moyen expiratoire n'est pratiquement pas réduite). Puis la pression chute et la bille revient à sa position initiale d'obstruction de l'orifice du cône, créant à nouveau une surpression déterminée, qui constitue le départ d'un nouveau cycle.

Un cycle d'ouverture/fermeture se compose donc d'un temps total d'ouverture TTO (= temps d'ouverture TO + temps d'ouverture totale TOT + temps de fermeture TF) et d'un temps d'interruption TI.

C'est pendant le temps d'interruption TI qu'est générée la surpression. Les phases d'accélération TO et d'ouverture totale TOT permettent de maintenir une très grande vitesse du flux d'air, ce qui, en plus de l'effet thérapeutique recherché, facilite grandement l'utilisation de l'appareil.

Durant le cycle expiratoire, la bille subit donc un mouvement oscillatoire. La fréquence des oscillations, c'est-à-dire le nombre de cycles d'ouverture/fermeture par unité de temps, peut être modulée par l'inclinaison de l'appareil de quelques degrés en-dessus ou en-dessous de l'horizontale.

On s'est également aperçu que lorsque cet appareil de résistance à l'expiration est utilisé par un patient immédiatement après l'inhalation d'un médicament, le flux d'air turbulent provoqué dans les poumons lors de l'expiration dans l'appareil permet d'une part aux particules de médicament de pénétrer plus loin dans l'arbre bronchique et d'autre part d'améliorer la déposition de ces particules dans les poumons.

Le but de la présente invention est de mettre à profit les observations qui précèdent et de proposer un appareil spécifique du domaine respiratoire conçu selon le principe de base de l'appareil décrit ci-dessus, mais offrant des possibiltés thérapeutiques améliorées et/ou plus larges.

L'appareil selon l'invention est défini dans les revendications 1, 2 et 5.

Selon les modes de réalisation définis dans les revendications 1 et 2, l'appareil permet d'obtenir une ventilation en percussion ayant les effets décrits plus haut, tant à l'expiration qu'à l'inspiration.

Selon un autre mode d'exécution défini dans la revendication 5, l'appareil est agencé pour permettre non seulement une ventilation pulmonaire en percussion, mais également pour favoriser la prise de médicaments.

Des modes de réalisation particuliers sont donnés aux revendications 3, 4 et 6 à 11.

La description qui suit, donnée à titre d'exemple, se réfère aux dessins annexés sur lesquels:
la figure 1 est une coupe verticale schématique d'un exemple d'appareil selon l'invention à double effet, tant à l'expiration qu'à l'inspiration;
la figure 2 est une coupe verticale schématique d'un exemple d'appareil selon l'invention muni d'une soupape d'inspiration, notamment pour la prise d'un médicament;
la figure 3 est une coupe verticale d'un détail d'une variante de soupape d'inspiration;
la figure 4 est une coupe verticale schématique d'un exemple d'appareil selon l'invention pouvant être connecté tant à un aérosol qu'à un spray-doseur;
la figure 5 est une vue depuis l'avant de l'appareil de la figure 4;
la figure 6 est une coupe verticale schématique d'un autre exemple d'appareil à double effet, combiné avec un aérosol-doseur;
la figure 7 est une coupe verticale schématique d'un autre exemple d'appareil à double effet,
la figure 8 est une coupe verticale schématique d'un exemple d'appareil comportant une chambre de nébulisation,
la figure 9 est une coupe verticale schématique d'un autre exemple d'appareil muni d'un spray-doseur,
la figure 10 est une coupe verticale schématique d'une variante de l'appareil de la figure 4,
la figure 11 est un diagramme illustrant le principe du cycle d'ouverture/fermeture d'un appareil de résistance à l'expiration, tel que celui décrit dans le brevet européen no 0337990; et
les figures 12a à 12d illustrent différentes formes d'éléments de cônes permettant une adaptation du cycle d'ouverture/fermeture à différents modes thérapeutiques spécifiques.

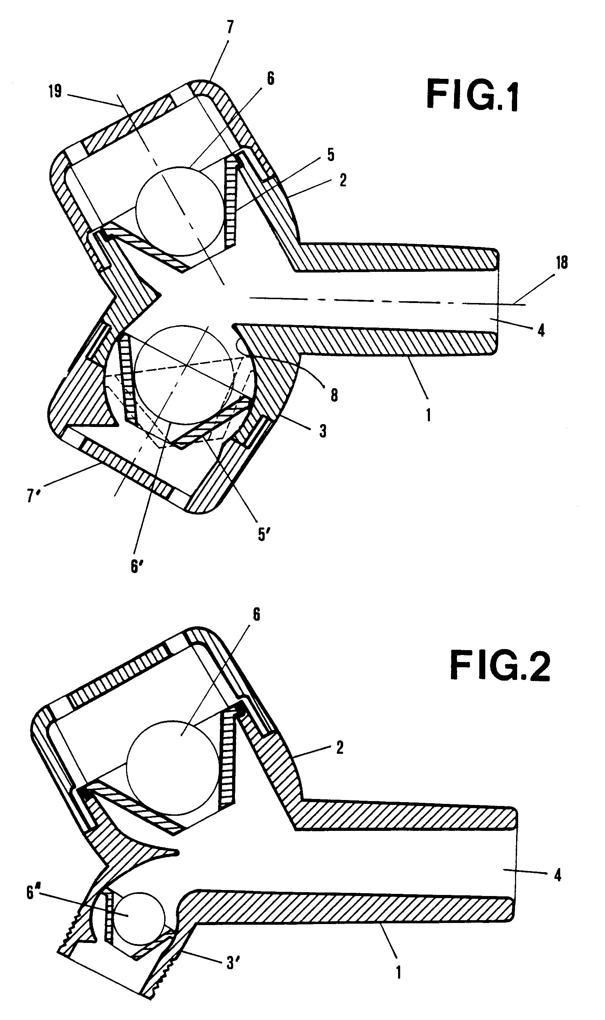

L'appareil représenté à la figure 1 est un appareil à double effet permettant d'obtenir une ventilation pulmonaire en percussion tant à l'expiration qu'à l'inspiration. Il comprend un élément en forme de double pipe, comportant une première partie 1 tubulaire rectiligne, une deuxième partie 2 inclinée vers le haut par rapport à la première partie et une troisième partie 3 inclinée vers le bas par rapport à la première partie. La première partie tubulaire comporte un orifice 4 d'entrée d'air dans lequel le patient peut soit expirer, soit inspirer. Chacune des deuxième et troisième parties comporte un élément 5, respectivement 5', en forme de cône tronqué et une bille 6, respectivement 6', dont le diamètre est supérieur au plus petit diamètre de l'élément conique correspondant. Le cône 5 de la partie inclinée vers le haut est disposé de façon que son orifice de plus grand diamètre soit situé vers le haut, et que la bille s'appuie à l'intérieur du cône sur les parois de celui-ci, les caractéristiques de cette partie de l'appareil étant celles de l'appareil décrit dans le brevet européen no 0337990 mentionné plus haut. De même, le cône 5' disposé dans la partie inclinée vers le bas est tourné de façon que son orifice de plus grand diamètre soit également situé vers le haut, c'est-à -dire dans ce cas vers l'intérieur de l'appareil, afin, qu'à l'état de repos, la seconde bille s'appuie sur les parois internes du cône et obstrue le canal. L'appareil comporte en outre deux couvercles 7, 7' disposés respectivement à l'extrémité de chacune des parties 2, 3 inclinées vers le haut et vers le bas, lesdits couvercles étant troués de façon à permettre d'une part à l'air expiré de s'échapper à travers le premier couvercle 7 et d'autre part à l'air inspiré de s'introduire à travers le second couvercle 7'. Il résulte de l'agencement décrit ci-dessus que, lors d'une expiration, l'orifice constitué par la partie inclinée vers le bas est fermé par le blocage de la seconde bille 6' dans le cône inférieur 5' sous l'effet de la pression de l'air, alors que l'air s'échappe par la partie inclinée vers le haut, la première bille 6 se déplaçant sous l'effet de l'expiration et procurant l'effet de ventilation en percussion déjà décrit plus haut. Lors d'une inspiration l'orifice constitué par la partie inclinée vers le haut est obstrué par la première bille 6 sous l'effet de son poids propre, alors que la bille 6' disposée dans le cône inférieur se soulève de façon à permettre le passage de l'air, d'où il résulte une ventilation en percussion du même type que celle qui intervient lors de l'expiration. Les couvercles 7, 7', ainsi que les cônes 5, 5' sont amovibles, de façon à permettre, notamment, un nettoyage efficace de l'appareil.

Le cône 5' de la partie inclinée vers le bas peut être incliné dans le même sens que le cône supérieur, de façon que son axe soit sensiblement parallèle à celui du cône supérieur, comme représenté en traitillé sur la figure 1. Il peut également être incliné dans le sens opposé au cône supérieur, comme représenté en trait plein sur la figure 1. Les angles d'inclinaison des cônes seront choisis de façon à éviter que les billes ne se soulèvent et flottent au-dessus de leur support, mais qu'elles restent en contact avec celui-ci, de façon à obtenir le meilleur effet de percussion souhaité. Selon le mode d'exécution représenté à la figure 1, le cône inférieur est monté en pivotement à l'intérieur d'un élément de sphère 8, ce qui permet de faire varier l'inclinaison du cône, en agissant par exemple sur un organe d'actionnement, tel qu'un axe (non-représenté) faisant saillie à l'extérieur de l'appareil.

La partie 2 inclinée vers le haut et la partie 3 inclinée vers le bas de l'appareil à double effet représenté à la figure 1 sont sensiblement de mêmes dimensions. On remarquera toutefois qu'une telle similitude de dimensions n'est nullement nécessaire et que la partie inclinée vers le bas peut être réalisée avec des dimensions inférieures (ou respectivement supérieures) à celles de la partie inclinée vers le haut et donc avec un cône et une bille de dimension inférieure (ou respectivement supérieure) à celle du cône et de la bille de la partie supérieure, selon la ou les fonction(s) assignée(s) à l'appareil et/ou l'effet thérapeutique recherché.

Il est d'autre part possible de réaliser l'appareil, comme représenté à la figure 2, avec une bille inférieure 6'' légère, l'appareil ne procurant pas lors d'une inspiration l'effet de percussion de l'appareil de la figure 1, mais seulement un effet de valve permettant, notamment, la prise d'un médicament. La bille inférieure 6'' de l'appareil représenté à la figure 2 est de dimension inférieure à celle de la bille supérieure 6. Il est bien entendu également possible de réaliser le même type d'appareil avec une bille inférieure légère, mais de dimension supérieure à celle de la bille supérieure. Dans les deux cas, on disposera de préférence le cône de support de la bille inférieure 6'' de façon que son axe soit incliné, afin que la bille ne se soulève pas, mais reste en contact avec le cône. En effet, si l'axe du cône est vertical, la bille se soulève et a tendance à effectuer des mouvements décrivant des circonvolutions le long des parois du cônes, ce qui freine le mouvement de fermeture. Par contre, lorsque l'axe du cône est incliné de façon que la bille reste en contact avec le cône et que le cône constitue un chemin de roulement pour la bille, le mouvement de fermeture est plus rapide.

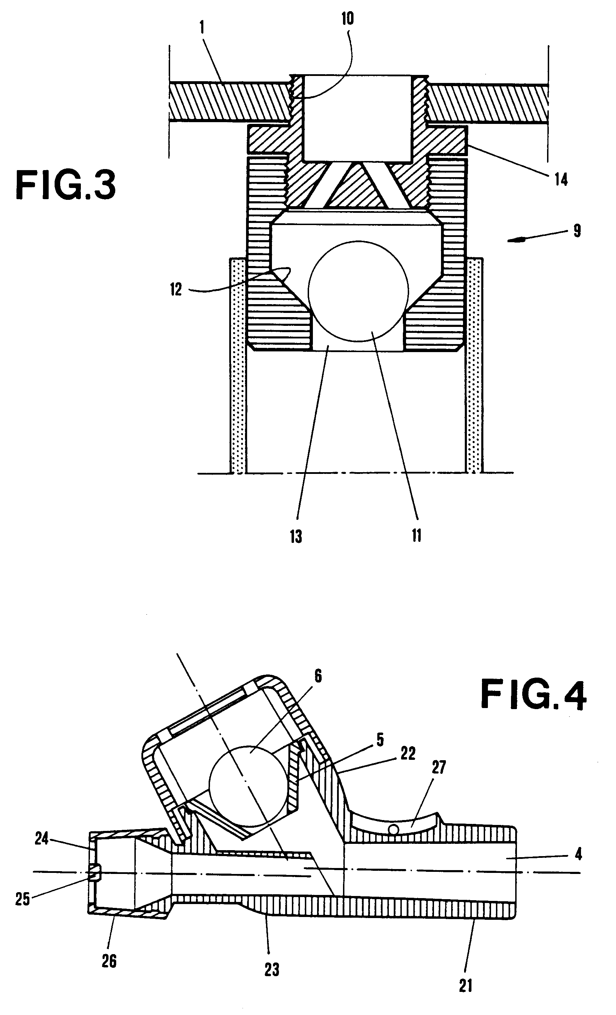

Selon une variante d'exécution de l'appareil de la figure 2, la partie 3' inclinée vers le bas peut être remplacée par un simple orifice 10 pratiqué dans la partie inférieure de la première partie tubulaire rectiligne 1, à proximité de la partie coudée. Un dispositif 9 permettant la prise d'un médicament lors d'une inspiration est monté sur l'appareil à l'endroit de cet orifice, par exemple par vissage. Comme représenté à la figure 3, ce dispositif comporte une valve d'inspiration, fixée sur l'appareil par l'intermédiaire d'un raccord à évent 14. La valve est agencée pour être fermée à l'état de repos ou lors d'une expiration, mais pour s'ouvrir lors d'une inspiration. La valve comporte une bille 11 qui repose à l'état de repos sur le bord conique 12 d'une ouverture 13 du dispositif de prise de médicaments. Bien entendu, toute autre forme adéquate de valve pourrait être prévue.

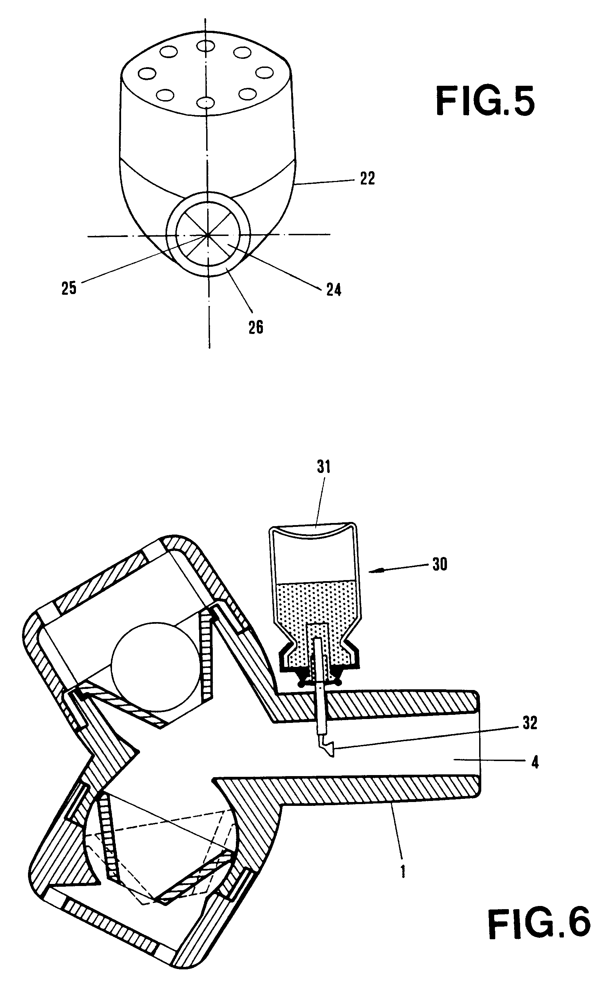

L'appareil représenté schématiquement aux figures 4 et 5 comporte les éléments traditionnels déjà décrits permettant la ventilation pulmonaire en percussion lors d'une expiration du patient. La partie tubulaire rectiligne 21 qui constitue l'embout de l'appareil est prolongée à son autre extrémité, après la partie 22 inclinée vers le haut, par une portion d'élément tubulaire rectiligne 23 dont l'axe est parallèle à celui de l'embout. Cette portion peut comporter une valve 24 à son extrémité, par exemple sous forme d'un disque de caoutchouc souple fixé en son centre 25 à l'extrémité d'un élément en forme de tube légèrement conique 26. L'élément 26 est monté, par exemple par clipsage, à l'extrémité avant de la portion d'élément tubulaire 23. La forme conique de l'élément 26 permet d'y fixer un tuyau relié à un aérosol compresseur ou à un spray-doseur, en introduisant simplement l'extrémité du tuyau autour de la partie 26 et en forçant. Le même montage peut être réalisé sur l'élément à soupape des figures 2 et 3. L'appareil peut comporter un niveau 27 permettant de contrôler son inclinaison. En outre, une chambre du type "spacer" peut être insérée entre l'appareil et le tuyau de façon à créer une réserve de produit à inhaler.

La figure 6 illustre un appareil à double effet combiné avec un aérosol-doseur 30 monté à la partie supérieure de la partie d'embout 1, l'aérosol-doseur étant agencé de façon qu'une pression sur sa base 31 injecte une dose 32 de médicament à l'intérieur de l'embout 4.

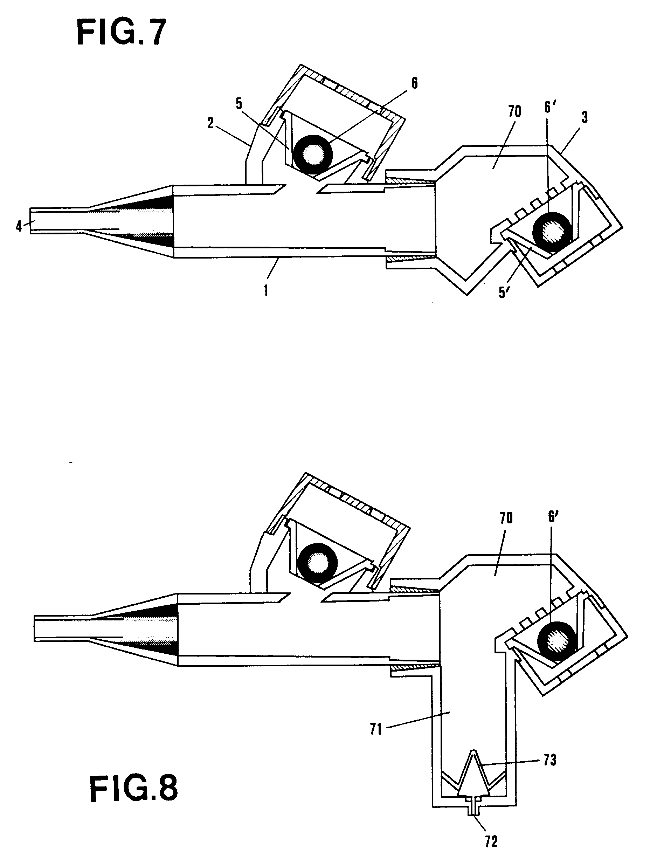

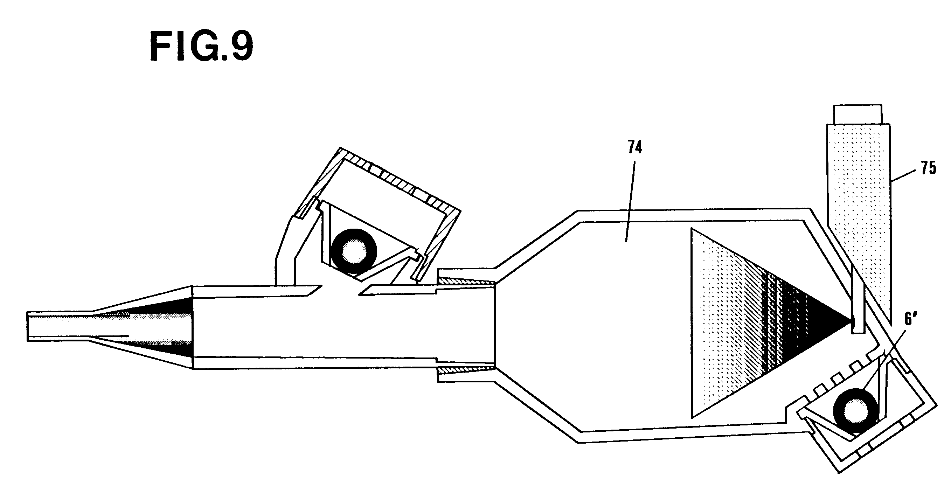

L'appareil représenté à la figure 7 est un appareil à double effet permettant d'obtenir les mêmes effets que l'appareil représenté à la figure 1. On retrouve comme à la figure 1 une première partie 1 tubulaire rectiligne, une deuxième partie 2 inclinée vers le haut et une troisième partie 3 inclinée vers le bas. Toutefois la troisième partie est décalée vers l'avant par rapport à la deuxième partie et montée de façon amovible, par exemple par simple emboîtement conique, à l'extrémité de la première partie, de façon à constituer un appareil modulaire. Cette conception modulaire facilite d'une part le démontage de l'appareil pour son nettoyage et permet d'autre part de modifier facilement l'appareil pour l'obtention par exemple d'une des configuration représentées sur les figures 8 et 9.

Ainsi, selon la configuration de l'appareil représenté à la figure 8, la troisième partie comprend un espace 70 agrandi par rapport à celui de l'appareil de la figure 7, de façon à constituer, par exemple, une chambre de nébulisation 71, munie d'un orifice 72 permettant sa connexion à un compresseur. Comme représenté à la figure 8, le nébulisateur 73 peut être logé à l'intérieur de la chambre 71.

De même, selon la configuration représentée à la figure 9, la troisième partie de l'appareil comprend une chambre 74, sur laquelle est monté un spray-doseur 75, ladite chambre 74 constituant ce que l'on appelle dans le langage consacré un "spacer", destiné à mettre à disposition du patient un volume de particules plus important et plus homogène et à créer une réserve de produit à inhaler, permettant une meilleure prise des particules par le patient.

Bien entendu, la bille 6' de l'un quelconque des appareils représentés aux figures 7 à 9 peut être une bille légère, qui n'a alors plus d'autre fonction que celle d'une valve.

On notera encore qu'il est possible de remplacer l'embout buccal de chacun des appareils décrits ci-dessus par un dispositif de connexion permettant l'intégration de l'appareil dans un système d'assistance respiratoire.

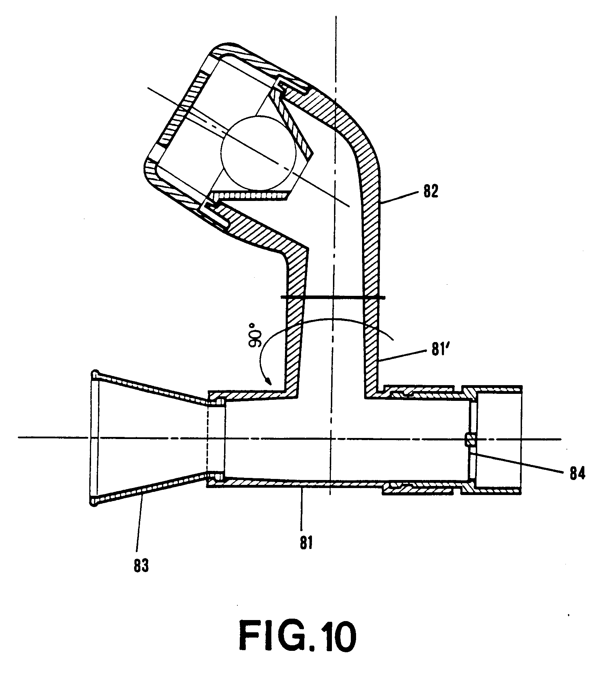

L'appareil représenté à la figure 10 est une variante de l'appareil de la figure 4. Il comporte un élément tubulaire 81, 81' en forme de T muni d'un élément 82 de résistance à l'expiration, et comportant un embout buccal 83 à l'une des ses extrémités latérales et une soupape 84 à son extrémité opposée. Pour des raisons de représentation, on a représenté l'élément 82 dans le même plan que l'élément tubulaire en forme de T. En réalité, l'élément tubulaire 81 se trouve être perpendiculaire au plan du dessin.

Afin de permettre au thérapeute d'affiner son traitement en fonction des paramètres du patient à traiter, l'élément de cône 5, s'il s'agit d'un appareil à simple effet à l'expiration, et/ou l'élément de cône 5', lorsqu'il s'agit d'un appareil à double effet, peuvent être conformés de façon que la génératrice du cône ait une forme (comme représenté par exemple sur l'une des figures 12a à 12d) déterminée en fonction d'un cycle particulier d'ouverture du canal. De même, afin de faire évoluer le traitement pour un patient donné, chacun des appareils décrits ci-dessus peut être réalisé avec un canal d'échappement d'air, et/ou le cas échéant, avec un canal d'inspiration d'air, comportant un élément de cône amovible, un ensemble d'éléments de cône interchangeables étant prévu, chacun desdits éléments de cône ayant une forme agencée pour correspondre à un cycle particulier d'ouverture/fermeture du canal (figure 11).

Différentes formes de cônes peuvent être prévues selon l'effet souhaité. Les diverses formes intéressantes peuvent être regroupées en quatre groupes, que l'on a représenté sur les figures 12a à 12d.

Le premier groupe concerne des éléments de cône 40 ayant une génératrice de forme concave (figure 12a). Une telle forme permet de diminuer la phase de surpression (temps d'interruption TI), d'augmenter le temps d'ouverture, ainsi que la fréquence des oscillations et éventuellement le temps d'ouverture totale et de diminuer l'accélération du flux.

Le deuxième groupe concerne des éléments de cône 41 ayant une génératrice de forme convexe (figure 12b). Une telle forme permet d'augmenter la phase de surpression (augmentation du temps TI), de diminuer le temps d'ouverture, ainsi que la fréquence des oscillations, tout en augmentant le temps d'ouverture totale et l'accélération du flux.

Le troisième groupe concerne des éléments de cône 42 dont la génératrice comprend une partie inférieure de forme convexe et une partie supérieure de forme concave (figure 12c).

Le quatrième groupe comprend des éléments de cône 43 dont la génératrice comprend une partie inférieure de forme concave et une partie supérieure de forme convexe (figure 12d).

D'autres formes ou combinaisons de formes peuvent bien entendu être envisagées dans le cadre des revendications.

## Revendications

1. Appareil thérapeutique spécifique du domaine respiratoire comportant une première partie (1) de forme tubulaire comportant un orifice d'entrée d'air (4) dans lequel le patient peut expirer et une seconde partie comportant des moyens pour engendrer une ventilation pulmonaire variable, notamment un effet de percussion, lors de l'expiration du patient, lesdits moyens comportant un canal d'échappement (5) de forme conique, dans lequel est disposée une bille sphérique (6) de diamètre supérieur aux dimensions de l'orifice d'entrée du canal, de façon à obstruer le canal avant expiration, l'axe (19) du canal conique étant incliné vers le haut par rapport à l'axe (18) de la partie de forme tubulaire, l'angle formé par une génératrice de la paroi de ce canal et son axe étant inférieur à l'angle d'inclinaison de l'axe du canal, l'appareil étant conformé de façon à permettre à l'air expiré de s'échapper par au moins un trou situé dans une zone opposée à l'orifice d'entrée dudit canal, caractérisé en ce qu'il comporte au moins un troisième orifice comportant un dispositif d'ouverture/fermeture agencé pour s'ouvrir lors d'une inspiration du patient comportant des moyens (3, 5', 6') pour engendrer une ventilation pulmonaire variable lors de l'inspiration du patient, lesdits moyens étant du même type que les moyens permettant la ventilation en percussion lors d'une expiration.

2. Appareil thérapeutique spécifique du domaine respiratoire comportant une première partie (1) de forme tubulaire comportant un orifice d'entrée d'air (4) dans lequel le patient peut expirer et une seconde partie comportant des moyens pour engendrer une ventilation pulmonaire variable, notamment un effet de percussion, lors de l'expiration du patient, lesdits moyens comportant un canal d'échappement (5) de forme conique, dans lequel est disposée une bille sphérique (6) de diamètre supérieur aux dimensions de l'orifice d'entrée du canal, de façon à obstruer le canal avant expiration, l'axe (19) du canal conique étant incliné vers le haut par rapport à l'axe (18) de la partie de forme tubulaire, l'angle formé par une génératrice de la paroi de ce canal et son axe étant inférieur à l'angle d'inclinaison de l'axe du canal, l'appareil étant conformé de façon à permettre à l'air expiré de s'échapper par au moins un trou situé dans une zone opposée à l'orifice d'entrée dudit canal, caractérisé en ce qu'il comporte au moins un troisième orifice comportant un dispositif d'ouverture/fermeture agencé pour s'ouvrir lors d'une inspiration du patient et comportant un canal d'inspiration de forme conique, dans lequel est disposée une bille sphérique (6'') de diamètre supérieur aux dimensions de l'orifice d'entrée du canal d'inspiration, l'ensemble étant agencé de façon que la bille obstrue le canal d'inspiration avant l'inspiration.

3. Appareil selon l'une des revendications 1 ou 2, caractérisé en ce que le dispositif d'ouverture/fermeture agencé pour s'ouvrir lors d'une inspiration du patient comporte des moyens pour sa connexion à un dispositif dispenseur de médicament.

4. Appareil selon l'une des revendications 1 ou 2, caractérisé en ce qu'il comporte un spray-doseur monté sur l'appareil, l'ensemble étant agencé pour l'injection d'un médicament dans l'appareil.

5. Appareil thérapeutique spécifique du domaine respiratoire comportant une première partie (1) de forme tubulaire comportant un orifice d'entrée d'air (4) dans lequel le patient peut expirer et une seconde partie comportant des moyens pour engendrer une ventilation pulmonaire variable, notamment un effet de percussion, lors de l'expiration du patient, lesdits moyens comportant un canal d'échappement (5) de forme conique, dans lequel est disposée une bille sphérique (6) de diamètre supérieur aux dimensions de l'orifice d'entrée du canal, de façon à obstruer le canal avant expiration, l'axe (19) du canal conique étant incliné vers le haut par rapport à l'axe (18) de la partie de forme tubulaire, l'angle formé par une génératrice de la paroi de ce canal et son axe étant inférieur à l'angle d'inclinaison de l'axe du canal, l'appareil étant conformé de façon à permettre à l'air expiré de s'échapper par au moins un trou situé dans une zone opposée à l'orifice d'entrée dudit canal, caractérisé en ce qu'il comporte au moins un troisième orifice comportant un dispositif d'ouverture/fermeture agencé pour s'ouvrir lors d'une inspiration du patient, ledit dispositif d'ouverture/fermeture étant agencé pour la prise d'un médicament, l'appareil permettant d'associer l'effet résultant de la ventilation pulmonaire variable, notamment l'effet de percussion, à la prise du médicament et à améliorer celle-ci.

6. Appareil selon la revendication précédente, caractérisé en ce que le dispositif d'ouverture/fermeture à l'inspiration comporte une valve.

7. Appareil selon l'une des revendications 5 ou 6, caractérisé en ce que le troisième orifice comporte des moyens pour sa connexion à un dispositif dispenseur de médicament.

8. Appareil selon la revendication 7, caractérisé en ce que le dispositif dispenseur de médicament est un aérosol ou un spray-doseur.

9. Appareil selon l'une des revendications 5 à 8, caractérisé en ce que le dispositif d'ouverture/fermeture à l'inspiration comporte un canal d'inspiration de forme conique, dans lequel est disposé une bille sphérique (6'; 6'') de diamètre supérieur aux dimensions de l'orifice d'entrée du canal, de façon à obstruer le canal avant l'inspiration.

10. Appareil selon l'une des revendications précédentes, caractérisé en ce qu'il est conformé de façon que les dispositifs d'ouverture à l'expiration et à l'inspiration sont séparés par un espace ou volume interne (70, 71, 74).

11. Appareil selon la revendication 10, caractérisé en ce que ledit espace interne constitue un espace de mélange et de réserve.

## Claims

1. A therapeutic apparatus for specific use in the respiratory field comprising a first part (1) having a tubular shape and comprising an air inlet orifice (4) into which the patient may exhale and a second part comprising means for producing a variable pulmonary ventilation, in particular a percussion effect, when the patient expires, said means comprising an outlet duct (5) having a conical shape, in which is disposed a spherical ball (6) having a diameter larger than the dimensions of the inlet orifice of the duct, in order to obstruct the duct before expiration, the axis (19) of the conical duct being upwardly inclined with respect to the axis (18) of the tubular-shaped part, the angle formed by a generator of the wall of this duct and its axis being less than the angle of inclination of the axis of the duct, the apparatus being designed so as to enable expired air to escape through at least one hole situated in a zone opposite the inlet orifice of said duct, characterised in that it comprises at least a third orifice comprising an opening/closing device designed to open during an inspiration of the patient comprising means (3, 5', 6') for producing a variable pulmonary ventilation during the inspiration of the patient, said means being of the same type as the means enabling percussive ventilation during expiration.

2. A therapeutic apparatus for specific use in the respiratory field comprising a first part (1) having a tubular shape and comprising an air inlet orifice (4) into which the patient may exhale and a second part comprising means for producing a variable pulmonary ventilation, in particular a percussion effect, when the patient expires, said means comprising an outlet duct (5) having a conical shape, in which is disposed a spherical ball (6) having a diameter larger than the dimensions of the inlet orifice of the duct, in order to obstruct the duct before expiration, the axis (19) of the conical duct being upwardly inclined with respect to the axis (18) of the tubular-shaped part, the angle formed by a generator of the wall of this duct and its axis being less than the angle of inclination of the axis of the duct, the apparatus being designed so as to enable expired air to escape through at least one hole situated in a zone opposite the inlet orifice of said duct, characterised in that it comprises at least a third orifice comprising an opening/closing device designed to open during an inspiration of the patient and comprising an inspiration duct having a conical shape, in which is disposed a spherical ball (6'') having a diameter which is greater than the dimensions of the inlet orifice of the inspiration duct, the whole being arranged so as the ball obstructs the inspiration duct before inspiration.

3. An apparatus according to one of the claims 1 or 2, characterised in that the opening/closing device arranged to open during an inspiration of the patient comprises means for its connection to a medication dispenser device.

4. An apparatus according to one of the claims 1 or 2, characterised in that it comprises a spray metering device mounted on the apparatus, the unit being designed for the injection of a medication into the apparatus.

5. A therapeutic apparatus for specific use in the respiratory field comprising a first part (1) having a tubular shape and comprising an air inlet orifice (4) into which the patient may exhale and a second part comprising means for producing a variable pulmonary ventilation, in particular a percussion effect, when the patient expires, said means comprising an outlet duct (5) having a conical shape, in which is disposed a spherical ball (6) having a diameter larger than the dimensions of the inlet orifice of the duct, in order to obstruct the duct before expiration, the axis (19) of the conical duct being upwardly inclined with respect to the axis (18) of the tubular-shaped part, the angle formed by a generator of the wall of this duct and its axis being less than the angle of inclination of the axis of the duct, the apparatus being designed so as to enable expired air to escape through at least one hole situated in a zone opposite the inlet orifice of said duct, characterised in that it comprises at least a third orifice comprising an opening/closing device designed to open during an inspiration of the patient, said opening/closing device being arranged for the taking of a medication, the apparatus enabling to associate the effect resulting from the variable pulmonary ventilation, in particular the percussion effect, with the taking of the medication and to improve the same.

6. An apparatus according to the preceding claim, characterised in that the inspiration opening/closing device comprises a valve.

7. An apparatus according to one of the claims 5 or 6, characterised in that the third orifice comprises means for its connection to a medication dispenser device.

8. An apparatus according to claim 7, characterised in that the medication dispenser device is an aerosol or a spray metering device.

9. An apparatus according to one of the claims 5 to 8, characterised in that the inspiration opening/closing device comprises an inspiration duct having a conical shape, in which is disposed a spherical ball (6', 6'') having a diameter which is greater than the dimensions of the inlet orifice of the duct, so as to obstruct the duct before inspiration.

10. An apparatus according to one of the preceding claims, characterised in that the devices which open during expiration and inspiration are separated by an internal space or volume (70, 71, 74).

11. An apparatus according to claim 10, characterised in that said internal space constitutes a mixing and reserve space.

## Patentansprüche

1. Therapeutischer Apparat für das Gebiet der Beatmungstechnik, welcher ein erstes röhrenförmiges Teil (1) mit einer Lufteingangsöffnung (4) aufweist, in die der Patient ausatmen kann, sowie ein zweites Teil mit Einrichtungen, um während des Ausatmens des Patienten eine variable Lungenventilation, insbesondere einen Perkussionseffekt, zu erzeugen, wobei die besagten Einrichtungen einen konischen Ausströmkanal (5) aufweisen, in dem eine Kugel (6), deren Durchmesser größer als die Abmessungen der Eingangsöffnung des Kanals ist, derart angeordnet ist, daß der Kanal vor der Ausatmung versperrt wird, wobei die Achse (19) des konischen Kanals im Verhältnis zu der Achse (18) des röhrenförmigen Teiles nach oben geneigt ist und der Winkel zwischen einer Erzeugenden der Wand dieses Kanals und seiner Achse kleiner als der Neigungswinkel der Achse des Kanals ist, und der Apparat so ausgestaltet ist, daß die ausgeatmete Luft durch zumindest ein in einer der Eingangsöffnung des besagten Kanals gegenüberliegenden Zone befindliches Loch ausströmen kann, dadurch gekennzeichnet, daß er zumindest eine dritte Öffnung mit einer Öffnungs/Schließeinrichtung aufweist, die während einer Einatmung des Patienten geöffnet wird, und die eine Anordnung (3, 5', 6') aufweist zur Erzeugung einer variablen Lungenventilation während des Einatmens des Patienten, wobei diese Anordnung von der gleichen Art wie die Anordnung ist, die die Perkussionsventilation während des Ausatmens bewirkt.

2. Therapeutischer Apparat für das Gebiet der Beatmungstechnik, welcher ein erstes röhrenförmiges Teil (1) mit einer Lufteingangsöffnung (4) aufweist, in die der Patient ausatmen kann, sowie ein zweites Teil mit Einrichtungen, um während des Ausatmens des Patienten eine variable Lungenventilation, insbesondere einen Perkussionseffekt, zu erzeugen, wobei die besagten Einrichtungen einen konischen Ausströmkanal (5) aufweisen, in dem eine Kugel (6), deren Durchmesser größer als die Abmessungen der Eingangsöffnung des Kanals ist, derart angeordnet ist, daß der Kanal vor der Ausatmung versperrt wird, wobei die Achse (19) des konischen Kanals im Verhältnis zu der Achse (18) des röhrenförmigen Teiles nach oben geneigt ist und der Winkel zwischen einer Erzeugenden der Wand dieses Kanals und seiner Achse kleiner als der Neigungswinkel der Achse des Kanals ist, und der Apparat so ausgestaltet ist, daß die ausgeatmete Luft durch zumindest ein in einer der Eingangsöffnung des besagten Kanals gegenüberliegenden Zone befindliches Loch ausströmen kann, dadurch gekennzeichnet, daß er zumindest eine dritte Öffnung mit einer Öffnungs/Schließeinrichtung aufweist, die während einer Einatmung des Patienten geöffnet wird, sowie einen konischen Einatmungskanal aufweist, in dem eine Kugel (6'') angeordnet ist, deren Durchmesser größer ist als die Abmessungen der Einlaßöffnung des Einatmungskanals, so daß die Kugel den Einatmungskanal vor dem Einatmen verschließt.

3. Apparat nach einem der Ansprüche 1 oder 2, dadurch gekennzeichnet, daß die Öffnungs/Schließeinrichtung, die während einer Einatmung des Patienten geöffnet werden kann, eine Anordnung aufweist, um sie mit einer Vorrichtung zur Abgabe eines Medikaments zu verbinden.

4. Apparat nach einem der Ansprüche 1 oder 2, dadurch gekennzeichnet, daß er einen an ihm befestigten Spray-Dosierer aufweist zum Einsprühen eines Medikaments in den Apparat.

5. Therapeutischer Apparat für das Gebiet der Beatmungstechnik, welcher ein erstes röhrenförmiges Teil (1) mit einer Lufteingangsöffnung (4) aufweist, in die der Patient ausatmen kann, sowie ein zweites Teil mit Einrichtungen, um während des Ausatmens des Patienten eine variable Lungenventilation, insbesondere einen Perkussionseffekt, zu erzeugen, wobei die besagten Einrichtungen einen konischen Ausströmkanal (5) aufweisen, in dem eine Kugel (6), deren Durchmesser größer als die Abmessungen der Eingangsöffnung des Kanals ist, derart angeordnet ist, daß der Kanal vor der Ausatmung versperrt wird, wobei die Achse (19) des konischen Kanals im Verhältnis zu der Achse (18) des röhrenförmigen Teiles nach oben geneigt ist und der Winkel zwischen einer Erzeugenden der Wand dieses Kanals und seiner Achse kleiner als der Neigungswinkel der Achse des Kanals ist, und der Apparat so ausgestaltet ist, daß die ausgeatmete Luft durch zumindest ein in einer der Eingangsöffnung des besagten Kanals gegenüberliegenden Zone befindliches Loch ausströmen kann, dadurch gekennzeichnet, daß er zumindest eine dritte Öffnung mit einer Öffnungs/Schließeinrichtung aufweist, die während einer Einatmung des Patienten geöffnet wird, wobei die Öffnungs/Schließeinrichtung derart ausgebildet ist, daß sie die Einnahme eines Medikaments ermöglicht, so daß der Apparat die Verbindung des aus der variablen Lungenventilation resultierenden Effektes, insbesondere den Perkussionseffekt, mit der Einnahme verbindet, um deren Wirkung zu erhöhen.

6. Apparat nach dem vorhergehenden Anspruch, dadurch gekennzeichnet, daß die Öffnungs/Schließeinrichtung zur Einatmung ein Ventil aufweist.

7. Apparat nach einem der Ansprüche 5 oder 6, dadurch gekennzeichnet, daß die dritte Öffnung eine Vorrichtung aufweist, um sie mit einer Vorrichtung zur Medikamentenabgabe zu verbinden.

8. Apparat nach Anspruch 7, dadurch gekennzeichnet, daß die Vorrichtung zur Medikamentenabgabe ein Aerosol oder ein Spray-Dosierer ist.

9. Apparat nach einem der Ansprüche 5 bis 8, dadurch gekennzeichnet, daß die Öffnungs/Schließeinrichtung zur Einatmung einen konischen Einatmungskanal aufweist, in dem eine Kugel (6', 6'') angeordnet ist, deren Durchmesser größer ist als die Abmessungen der Einlaßöffnung des Kanals, um so den Kanal vor der Einatmung zu versperren.

10. Apparat nach einem der vorhergehenden Ansprüche, dadurch gekennzeichnet, daß er derart ausgebildet ist, daß die Einrichtungen zur Öffnung bei der Ausatmung und bei der Einatmung durch einen Innenraum oder ein Innenvolumen (70, 71, 74) getrennt sind.

11. Apparat nach Anspruch 10, dadurch gekennzeichnet, daß diese Innenraum einen Mischraum und Reserveraum darstellt.
